(19) Europäisches Patentamt
European Patent Office
Office européen des brevets

(11) Veröffentlichungsnummer: **0 265 668**
**B1**

(12) **EUROPÄISCHE PATENTSCHRIFT**

(45) Veröffentlichungstag der Patentschrift: **09.01.91**

(51) Int. Cl.$^5$: **C 07 C 263/10**

(21) Anmeldenummer: **87113871.5**

(22) Anmeldetag: **23.09.87**

(54) **Verfahren zur Herstellung eines Diisocyanats.**

(30) Priorität: **03.10.86 DE 3633712**

(43) Veröffentlichungstag der Anmeldung:
**04.05.88 Patentblatt 88/18**

(45) Bekanntmachung des Hinweises auf die Patenterteilung:
**09.01.91 Patentblatt 91/02**

(84) Benannte Vertragsstaaten:
**BE DE FR GB IT NL**

(56) Entgegenhaltungen:
**DE-A-1 618 798**
**DE-A-1 903 579**

(73) Patentinhaber: **BAYER AG**
**D-5090 Leverkusen 1 Bayerwerk (DE)**

(72) Erfinder: **Schmidt, Manfred, Dr.**
**Zeisigstrasse 5**
**D-4047 Dormagen (DE)**
Erfinder: **König, Klaus, Dr.**
**Zum Hahnenberg 40**
**D-5068 Odenthal (DE)**
Erfinder: **Heitkämper, Peter, Dr.**
**Fliederweg 14**
**D-4047 Dormagen 11 (DE)**
Erfinder: **Pedain, Josef, Dr.**
**Haferkamp 6**
**D-5000 Köln 80 (DE)**

**EP 0 265 668 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein neues Verfahren zur Herstellung eines Esterdiisocyanats.

Estergruppen aufweisende Diisocyanate gehören zum bekannten Stand der Technik. Derartige Verbindungen werden beispielsweise in der DE—OS—1 618 798 beschrieben. Zur Herstellung dieser Estergruppen aufweisenden Diisocyanate geht man so vor, daß man Aminocarbonsäuren mit bestimmten Alkanolaminen verestert und die resultierenden Esterdiamine bzw. deren Hydrochloride mit Phosgen umsetzt. Als Aminocarbonsäure kann hierbei beispielsweise die aus ε-Caprolactam kostengünstig zugängliche 6-Aminocarpronsäure verwendet werden. Wie jedoch bereits aus der DE—OS—1 618 798 zu ersehen ist, ist der Einsatz der in dieser Veröffentlichung genannten Alkanolamine mit gewissen Nachteilen verbunden. Bei der Nacharbeitung der Beispiele 7 und 8 der DE—OS—1 618 798 (siehe nachstehende Vergleichsbeispiele Seite 1 und 2) zeigten sich dann zusätzliche Schwierigkeiten. So fallen die aus den bevorzugt genannten Alkanolaminen hergestellten Dihydrochloride als pastöse fest-flüssig Gemische an, die in den üblichen organischen Lösungsmitteln unlöslich sind. Die Isolierung—beispielsweise durch vorsichtiges Abziehen des Lösungsmittels—führt zu weiterer Zersetzung der Produkte, Hieraus erkennt man, daß eine Reinheitsprüfung der Dihydrochloride nicht möglich und insbesondere eine gegebenenfalls notwendige Reinigung undurchführbar ist. Eine veränderte Arbeitsweise zur Herstellung der Dihydrochloride z.B. nach H. Ulrich et al., Liebigs Ann. der Chemie, 1975, S. 1317, die als Ausgangsmaterial 6-Aminocaproylchlorid-hydrochlorid einsetzten, bringt keine Verbesserung. Auch nach dieser Methode fallen die Umsetzungsprodukte mit den in der DE—OS—1 618 798 bevorzugt genannten Alkanolamine als schmierige Produkte an, die weder aufzuarbeiten noch zu reinigen sind (nachstehendes Vergleichsbeispiel 3) und sich dementsprechend nicht zu sauberen Diisocyanaten umsetzen lassen.

Infolge der thermischen Instabilität der Dihydrochloride gestaltet sich auch der Austausch gegen das zur Phosgenierung benötigte Lösungsmittel sehr schwierig.

Die im Beispiel 8 der DE—OS—1 618 798 genannte Phosgenierung in o-Dichlorbenzol wird nicht wie üblich unter Rückflußbedingungen durchgeführt und man erhält dementsprechend Isocyanate mit hohen Gehalten an gebundenem Chlor, wie dies in diesem Beispiel auch erwähnt wird.

Jedoch auch die Verwendung eines niedrigsiedenden Lösungsmittels (nachstehendes Vergleichsbeispiel 3) führt bei Phosgenierung unter Rückflußbedingungen weder zu verwertbaren Ausbeuten noch zu sauberen Produkten, da die thermische Instabilität eine Reinigung unmöglich macht. Wird dagegen in o-Dichlorbenzol unter Rückfluß gearbeitet, führt dies nur zu Zersetzungsprodukten und nicht zu nennenswerten Isocyanatausbeuten.

In diesen Nachteilen dürfte auch der Grund zu sehen sein, weswegen die in der DE—OS—1 618 798 konkret beschriebenen Diisocyanate bislang keine technische Bedeutung erlangten.

Auch die DE—OS—1 903 579 beschreibt Esterdiisocyanate und Verfahren zu ihrer Herstellung, die der diesbezüglichen Offenbarung der DE—OS—1 618 798 weitgehend entsprechen, so daß die obengemachten Ausführungen auch für diese Vorveröffentlichung Gültigkeit haben.

Der vorliegenden Erfindung liegt nun die Aufgabe zugrunde, ein neues, Estergruppen aufweisendes Diisocyanat zur Verfügung zu stellen, dessen Herstellung nicht mit den genannten Nachteilen behaftet ist, d.h. welches aus wohlfeilen Ausgangsmaterialien in hohen Ausbeuten leicht zugänglich ist, und welches aufgrund seiner Eigenschaften gut als Isocyanatkomponente bei der Herstellung von Polyurethankunststoffen geeignet ist.

Diese Aufgabe konnte durch die Bereitstellung des nachstehend näher beschriebenen Verfahrens zur Herstellung eines neuen Esterdiisocyanats gelöst werden.

Gegenstand der Erfindung ist ein Verfahren zur Herstellung des Diisocyanats der Formel

$$OCN{-}(CH_2)_5{-}\overset{\overset{\displaystyle O}{\|}}{C}{-}O{-}CH_2{-}\underset{\underset{\displaystyle CH_3}{|}}{\overset{\overset{\displaystyle CH_3}{|}}{C}}{-}CH_2NCO$$

dadurch gekennzeichnet, daß man das Diamindihydrochlorid der Formel

$$\overset{\ominus}{Cl}\overset{\oplus}{H_3N}{-}(CH_2)_5{-}\overset{\overset{\displaystyle O}{\|}}{C}{-}O{-}CH_2{-}\underset{\underset{\displaystyle CH_3}{|}}{\overset{\overset{\displaystyle CH_3}{|}}{C}}{-}CH_2\overset{\oplus}{NH_3}\overset{\ominus}{Cl}$$

in an sich bekannter Weise mit Phosgen umsetzt.

Das erfindungsgemäß hergestellte Diisocyanat weist gegenüber den aus DE—OS—1 618 798 bekannten, Estergruppen aufweisenden Diisocyanaten insbesondere bezüglich seiner Herstellbarkeit entscheidende Vorteile auf. So führt der Einsatz von Neopentanolamin (3-Amino-2,2-dimethyl-propanol) bzw. dessen Hydrochlorids als Reaktionspartner für 6-Aminocaproylchloridhydrochlorid zu dem beim

erfindungsgemäßen Verfahren einzusetzenden Dihydrochlorid in praktisch analysenreiner Form (vgl. nachstehendes Beispiel 4). Infolge der kristallinen Konsistenz des erfindungsgemäßen Ausgangsmaterials ist es leicht handzuhaben und weiter zu verarbeiten.

Insbesondere kann darauf verwiesen werden, daß das Dihydrochlorid wegen seiner thermischen Belastbarkeit ohne weiteres in den zu Phosgenierung üblicherweise verwendeten Lösungsmitteln wie Chlorbenzol oder o-Dichlorbenzol unter Rückflußbedingungen phosgeniert werden kann. Diese, vorzugsweise unter Rückflußbedingungen unter Normaldruck ablaufende Phosgenierung in Chlorbenzol oder o-Chlorbenzol erfolgt im allgemeinen dergestalt, daß man in die unter Rückfluß befindliche Suspension des Dihydrochlorids kontinuierlich Phosgen einleitet, bis ein klare Lösung entsteht. Anschließend wird das Reaktionsgemisch destillativ von überschüssigem Phosgen und von Chlorbenzol befreit. Die Aufarbeitung des hierbei als Destillationsrückstand anfallenden, erfindungsgemäßen Diisocyanats kann durch Vakuumdestillation erfolgen (vgl. nachstehendes Beispiel 5).

Das erfindungsgemäße Diisocyanat stellt ein wertvolles Ausgangsmaterial zur Herstellung von Kunststoffen auf Isocyanatbasis nach dem Isocyanat-Polyadditionsverfahren dar, Hierzu kann das Diisocyanat sowohl als solches als auch in mit Blockierungsmitteln für Isocyanatgruppen wie z.B. ε-Caprolactam, Methyl-ethyl-ketoxim, Malonsäurediethylester oder Acetessigsäureethylester blockierter Form zum Einsatz gelangen. Das neue Diisocyanat kann selbstverständlich auch im Gemisch mit anderen Diisocyanaten der aus der Polyurethanchemie bekannten Art zum Einsatz gelangen. Wegen der stark ausgeprägten unterschiedlichen Reaktivität des asymmetrischen, erfindungsgemäßen Diisocyanats eignet es sich auch hervorragend zur Herstellung von Polyurethankunststoffen nach dem Zweistufenprinzip, wobei in einer ersten Stufe zunächst aus dem erfindungsgemäßen Diisocyanat und unterschüssigen Mengen an organischen Polyhydroxylverbindungen der in der Polyurethanchemie an sich bekannten Art freie Isocyanatgruppen aufweisende Prepolymere hergestellt werden, die anschließend in einer zweiten Reaktionsstufe mit geeigneten Kettenverlängerungsmitteln in das hochmolekulare Polyurethan überführt werden. Das erfindungsgemäße Diisocyanat kann auch vor seiner Verwendung als Aufbaukomponente für Polyurethankunststoffe in an sich bekannter Weise in ein Isocyanurat-, Uretdion- oder Biuretgruppen aufweisendes Derivat überführt werden, welches gegebenenfalls vor seiner Weiterverarbeitung destillativ von nicht umgesetztem Überschüssigem Diisocyanats befreit wird.

Das erfindungsgemäße Diisocyanat bzw. seine Modifizierungsprodukte können insbesondere auf dem Lacksektor, d.h. zur Herstellung von Ein- und Zweikomponenten-Polyurethanlacken Verwendung finden.

In den nachfolgenden Beispielen beziehen sich alle Prozentangaben auf Gewichtsprozente.

Vergleichsbeispiel 1
(entspricht Beispiel 7 der DE—OS—1 618 798)

In einem 2-1-Vierhalskolben mit Gaseinleitungsrohr und Rückflußkühler wurden 113,2 g (1,0 Mol) ε-Caprolactam und 1,0 Mol konzentrierte wäßrige Chlorwasserstoffsäure gegeben. Diese Lösung wurde unter Einleiten von Chlorwasserstoff (mit einer Geschwindigkeit von 0,5 Mol/Stunde) eine Stunde zum Rückfluß erhitzt, Danach wurden 89,0 g (1,0 mol) 2-Aminobutanol, das in 100 ml Benzol gelöst war, und 300 ml o-Dichlorbenzol im Verlauf einer halben Stunde zugesetzt. Während der Zugabe wurde die Zufuhr von gasförmigem Chlorwasserstoff auf 1 Mol pro Stunde erhöht. Anschließend wurde die Mischung weiter unter Rückfluß am Azeotropabscheider erhitzt, wobei beständig ein langsamer Chlorwasserstoffstrom eingeleitet wurde. Die Rückflußtemperatur lag konstant bei 115°C, wozu die periodische Zugabe von Benzol erforderlich war. Nach insgesamt 16,5 Stunden unter Rückfluß sammelte sich kein Wasser mehr in der Falle an. Dies diente als Hinweis darauf, daß die Veresterung abgeschlossen war. Eine genaue Analytik des so erhaltenen pastösen, festflüssigen Gemischs war jedoch nicht möglich. Der Versuch, einen Teil des Dihydrochlorids durch vorsichtiges Abziehen des Lösungsmittels zu isolieren, führte zur Zersetzung des Produkts.

Das Gemisch wurde bei 140°C vier Stunden und dann bei 150°C weitere 2 Stunden phosgeniert und zwar mit einem Phosgenstrom, der mit einer Geschwindigkeit von 2 Mol/Stunde zugeführt wurde. Das Benzol, welches in die Dean-Stark-Falle überging, mußte abgezogen werden, um die gewünschte Temperatur zu erreichen.

Als Reaktionsprodukt erhielt man eine teerartige Masse, von der das überstehende Lösungsmittel incl. Reaktionsprodukt abdekantiert wurde. Nach Abziehen des Lösungsmittels und Destillation über eine Dünnschichtkolonne (250°C/0,7 mbar) erhielt man 21 g (ca. 10% der Theorie) eines noch immer stark verunreinigten Produktes (nach gaschromatographischer Analyse).

Vergleichsbeispiel 2
(entsprechend Beispiel 8 der DE—OS—1 618 798)

6-Isocyanatocapronsäure-(2-isocyanato-2-methyl-propyl)-ester

In einem 2-1-Vierhalskolben mit Gaseinleitungsrohr und Rückflußkühler wurden 113 g ε-Caprolactam und 89 g 2-Amino-2-methylpropanol sowie nach und nach 2,2 Mol konzentrierte Chlorwasserstoffsäure gegeben. Nach 3 Stunden unter Rückfluß wurde die Hauptmenge des Wassers unter vermindertem Druck abdestilliert. Die Temperatur des Reaktionsgemisches wurde während des Destillierens unter 85°C gehalten. Dann wurden 450 ml o-Dichlorbenzol zugegeben, auf 115°C erhitzt und in langsamen Strom HCl-Gas eingeführt. Es wurden weiter 150 ml Benzol zugegeben und der letzte Rest Wasser azeotrop entfernt.

Auch hier diente nur das Ausbleiben einer weiteren Wasserabscheidung als Zeichen dafür, daß die Umsetzung abgeschlossen war. Eine genaue Analytik des sehr inhomogenen Reaktionsprodukts war nicht möglich. Das Benzol wurde dann unter vermindertem Druck abgezogen und durch 100 ml o-Dichlorbenzol ersetzt. Anschließend wurde dieses Gemisch 4 Stunden bei 130°C und dann 2 Stunden bei 140°C phosgeniert (200 bis 250 g/h Phosgen). Zum Entfernen von überschüssigem Phosgen wurde dann das klare Reaktionsgemisch 15 Minuten mit Stickstoff gespült, mit 500 ml wasserfreiem o-Dichlorbenzol versetzt und bei 80 bis 90°C und 15 mbar andestilliert (ca. 500 ml Destillat). Anschließend wurde das Gemisch abgekühlt, wobei sich eine viskose, bei Raumtemperatur harte Masse abschied, aus welcher durch Destillation wenige ml des—laut Gaschromatographie—stark verunreinigten Produktes abgetrennt werden konnte.

Vergleichsbeispiel 3

Herstellung von 6-Isocyanatocapronsäure-(2-isocyanato-2-methyl-1-propyl)-ester durch Phosgenierung in einem niedrigsiedenden Lösungsmittel.

In einem 4-1-Vierhalskolben wurden 92,2 g 2-Amino-2-methylpropanol in 1,000 ml Methylenchlorid vorgelegt. In diese Lösung wurde bis zur Sättigung bei 20 bis 40°C Chlorwasserstoffgas eingeleitet. Man erhielt eine Suspension des 2-Amino-2-methylpropanolhydrochlorids. In diese Mischung wurde eine Lösung von 186 g 6-Amino-caproylchloridhydrochlorid in 660 ml Methylenchlorid (Arbeitsweise nach H. Ulrich et al., Liebigs Ann der Chemie 1975, S. 1317) bei einer Temperatur von 20°C innerhalb von 50 Minuten zugegeben. Es bildete sich hierbei ein schmieriger Niederschlag. Danach wurde die Temperatur auf 40°C erhöht und dabei die restliche Chlorwasserstoffsäure ausgetrieben. Eine weitere Reinigung bzw. Isolierung des Dihydrochlorids war nicht möglich.

Zum Lösungsmittelaustausch wurden 1.700 ml Dichlorethan zugesetzt und das Methylenchlorid über eine 0,5 m Füllkörperkolonne abdestilliert. Eine direkte Herstellung des Dihydrochlorids in Dichlorethan war infolge der Schwerlöslichkeit von 6-Aminocaproylchloridhydrochlorid in diesem Lösungsmittel nicht möglich.

Das so erhaltene Diaminodihydrochlorid wurde in einer 4-1-Phosgenierapparatur nach Zugabe von einigen Siedesteinen unter Einleiten von Phosgen (50 g/h) zum Rückfluß erhitzt. Dabei verflüssigte sich das anfangs zähviskose Hydrochlorid, so daß das Rekationsgemisch gerührt werden konnte. Das Gemisch wurde 26 Stunden am Rückfluß (80°C) phosgeniert, wobei eine nahezu klare Lösung entstand. Anschließend wurde überschüssiges Phosgen durch Andestillieren entfernt und ein geringer Feststoffanteil durch Filtrieren abgetrennt. Die Lösung wurde anschließend destillativ vollständig von Dichlorethan befreit. Das zurückbleibende, bräunliche Rohisocyanat wurde in 6 Portionen im Vakuum destilliert, wobei 220 g fest farblose Flüssigkeit erhalten wurden. Zur Redestillation wurde das Produkt wieder in 6 Portionen geteilt, die jeweils bei 0,1 mbar und 98 bis 102°C destilliert wurden.

Gesamtausbeute: 210,5 g=83% der Theorie

ges. Destillationsrückstände: 33,9 g

farblose Flüssigkeit,

| | |
|---|---|
| NCO-Gehalt ber. | 33,0%, gef. 31,4% |
| hydr. Chlor: | 0,45% |

Nach massenspektrometrischen Untersuchungen bestand das Produkt nur zu ca. 78% aus dem gewünschten Diisocyanat, eine weitere Reinigung war nicht möglich, weil eine weitere Destillation des Gesamtansatzes an Redestillat bei ca. 100°C teilweise zu Zersetzung führte und daher abgebrochen werden mußte.

Beispiel 4

6-Aminocarponsäure-(3-amino-2,2-dimethyl-1-propyl)-ester-dihydrochlorid

In einem 10-1-Reaktionskolben wurden 535 g (5,2 Mol) 3-Amino-2,2-dimethylpropanol-1 in 5 l Methylenchlorid vorgelegt. In diese Lösung wurde bis zur Sättigung bei 20 bis 40°C HCl-Gas eingeleitet. Es resultierte eine leicht trübe Lösung des entsprechenden Neopentanolaminhydrochlorids. In diese Mischung wurde eine Lösung von 930 g (5 Mol) 6-Aminocaproylchlorid-hydrochlorid in 3,3 l $CH_2Cl_2$ (Herstellung nach H. Ulrich et al. Liebigs Ann. d. Chemie 1975, S. 1317—1321) bei einer Temperatur von 20°C innerhalb von 2 Stunden zugetropft.

Anschließend wurde die entstandene Salzsäure durch Erhitzen auf 40°C ausgetrieben. Der zunächst viskose Niederschlag ging hierbei in eine kristalline Form über und ließ sich problemlos absaugen. Nach Trocknen bei 50°C/100 mbar erhielt man ohne weitere Reinigung 1.420 g (98,3% der Theorie) des gewünschten Dihydrochlorids.

Schmelzpunkt: 154°C

$C_{11}H_{26}N_2O_2Cl_2$ (289)

| | |
|---|---|
| gef.: | C: 45,7%; H: 9,3%; N: 9,6%; Cl: 24,4% |
| Theorie: | C: 45,7%; H: 9,0%; N: 9,7%; Cl: 24,5% |

Beispiel 5

6-Isocyanatocapronsäure-(3-isocyanato-2,2-dimethyl-1-propyl)-ester

In einer 6-1-Laborphosgenierapparatur wurde ein Gemisch von 289 g feinkristallinem 6-Aminocapronsäure-(3-amino-2,2-dimethyl-1-propyl)-ester-dihydrochlorid und 3,5 l wasserfreiem Chlorbenzol vorgelegt und unter Rühren zügig zum Rückfluß erhitzt. Dabei wurde Phosgen in die Suspension eingeleitet (ca. 50 g/h). Das Gemisch wurde dann 8 Stunden am Rückfluß phosgeniert, wobei eine klare Lösung entstand. Anschließend wurde das Reaktionsgemisch destillativ von überschüssigem Phosgen und von Chlorbenzol befreit. Die zurückbleibende, bräunlich gefärbte Flüssigkeit wurde in Vakuum destilliert, wobei 6-Iso-cyanatocapronsäure-(3-isocyanato-2,2-dimethyl-1-propyl)-ester als farblose Flüssigkeit vom Siedepunkt 134 bis 138°C/0,1 mbar erhalten wurde. Das Diisocyanat ließ sich im gesamten Ansatz nahezu rückstandsfrei redestillieren.

$C_{13}H_{20}N_2O_4$ (268,3)
gef.: C: 58,1%; H: 7,6%; N: 10,5%
Theorie: C: 58,1%; H: 7,5%; N: 10,4%
NCO-Gehalt ber. 31,3% gef. 31,3%
Ausbeute: 252 g (94% der Theorie)
Siedepunkt: 115°C/0,05 mbar.

## Patentanspruch

Verfahren zur Herstellung des Diisocyanats der Formel

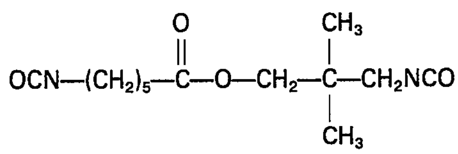

dadurch gekennzeichnet, daß man das Diamindihydrochlorid der Formel

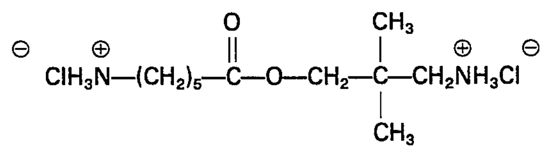

in an sich bekannter Weise mit Phosgen umsetzt.

## Revendication

Procédé de production du diisocyanate de formule

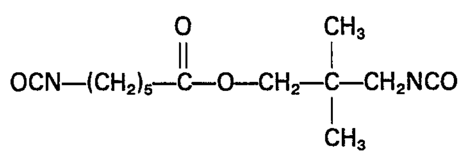

caractérisé en ce qu'on fait réagir d'une manière connue le dichlorhydrate de diamine de formule

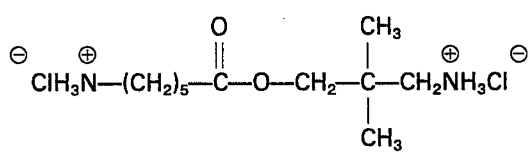

avec le phosgène.

**Claim**

A process for the production of a diisocyanate corresponding to the formula

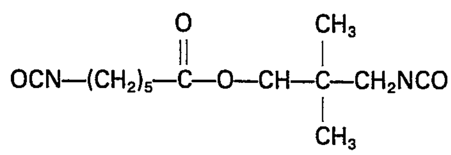

characterized in that a diamine dihydrochloride corresponding to the formula

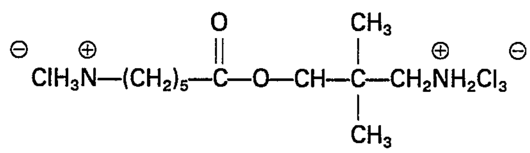

is reacted with phosgene by methods known *per se*.